# EUROPÄISCHE PATENTSCHRIFT

(11) **EP 0 968 431 B1**
(45) Veröffentlichungstag und Bekanntmachung des Hinweises auf die Patenterteilung: **28.08.2002**
(21) Anmeldenummer: 98916884.4
(22) Anmeldetag: 05.03.1998
(51) Int. Cl.: G01N 33/68

(54) **VERFAHREN ZUR SPEZIFISCHEN MARKIERUNG EINES PROTEINS**
METHOD FOR SPECIFICALLY MARKING A PROTEIN
PROCEDE DE MARQUAGE SPECIFIQUE D'UNE PROTEINE

(30) Priorität: 06.03.1997 DE 19709168; 29.10.1997 DE 19747632
(43) Veröffentlichungstag der Anmeldung: 05.01.2000
(73) Patentinhaber: Evotec OAI AG, 22525 Hamburg (DE); IHF INSTITUT FÜR HORMON- UND FORTPFLANZUNGSFORSCHUNG GmbH, 22529 Hamburg (DE)
(72) Erfinder: WILLEY, Kevin, D-22393 Hamburg (DE); OBERMANN-PLESS, Heike, D-21335 Lüneburg (DE); HUNT, Nicholas, D-21629 Neu-Wulmstorf (DE); HENCO, Karsten, D-40699 Erkrath (DE)
(74) Vertreter: Meyers, Hans-Wilhelm, Dr.Dipl.-Chem.
(86) Internationale Anmeldenummer: EP9801229
(87) Internationale Veröffentlichungsnummer: WO98039660

(56) Entgegenhaltungen:
- EP-A- 0 524 722
- US-A- 5 478 729
- FIRST E A ET AL: "SELECTIVE MODIFICATION OF THE CATALYTIC SUBUNIT OF CYCLIC AMP-DEPENDENT PROTEIN KINASE WITH SULFHYDRYL-SPECIFIC FLUORESCENT PROBES." BIOCHEMISTRY 28 (8). 1989. 3598-3605. CODEN: BICHAW ISSN: 0006-2960, XP002073139

## Beschreibung

Die vorliegende Erfindung betrifft ein Verfahren zur spezifischen Markierung eines Selenocyst(e)in- und/oder Cyst(e)ingruppen enthaltenden Proteins.

Die Untersuchung von Rezeptoren, wie z. B. den G-Protein-gekoppelten Rezeptoren, deren Polypeptidkette sich zu einer Struktur mit sieben membrandurchspannenden Helices faltet, ist sowohl wissenschaftlich als auch wirtschaftlich von großer Bedeutung. Es handelt sich bei diesen sogenannten 7-Transmembranrezeptoren um eine große Gruppe von Rezeptoren, die phylogenetisch sehr alt ist und deren Aminosäuresequenz charakteristische Cysteine aufweist. Die Liganden gehören dabei den unterschiedlichsten chemischen Stoffklassen und Molekülgrößen an, angefangen bei Lipiden, Metallionen und Monoaminen bis hin zu Peptiden und Proteinen. Über mögliche strukturelle Veränderungen derartiger Rezeptoren, welche das Bindeglied zwischen Ligandenbindung und Signaltransduktion darstellen, ist jedoch bislang nur wenig bekannt.

Es wird vermutet, daß ligandeninduzierte Konformationsänderungen der 7-Transmembranrezeptoren am Initialschritt der Dissoziation des heterotrimeren G-Protein-Komplexes unter Austausch von GDP/GTP beteiligt sind. Schwartz und Rosenkilde (TiPS, Vol. 17, 1996) stellen ein Modell vor, welches als einzige Anforderung an einen neuen Agonisten die Fähigkeit zur Stabilisierung einer aktiven Rezeptorkonformation nennt, und gelangen zu dem Schluß, daß kein gemeinsames "Schloß" für alle "Schlüssel" in der Superfamilie der 7-Transmembranrezeptoren existiert.

Allgemein werden bei der Untersuchung von Proteinen, aber auch insbesondere bei der Erforschung der Signalkaskade, Cystein-spezifische Reagenzien verwendet, um die funktionalen und strukturellen Eigenschaften der Cysteine näher zu beleuchten (Creighton, "Proteins", Freeman & Co., 1984).

Korner et al. (The Journal of Biological Chemistry, Vol. 257, No. 7, pp. 3389 - 3396, 1982) beschreiben ein Verfahren zur Untersuchung des β₂-adrenergen Rezeptors unter Verwendung von N-Ethylmaleimid (NEM). Um bereits exponierte Thiolgruppen vor dem eigentlichen Experiment zur Reaktion zu bringen, wurden die Membranproben in einem ersten Verfahrensschritt mit NEM in Gegenwart des Antagonisten Propranolol vorbehandelt. In einem weiteren Verfahrensschritt erfolgte eine Inkubation mit [³H]Isoproterenol unter Zusatz von NEM. Die Autoren kommen insbesondere zu dem Schluß, daß NEM nicht mit dem Rezeptor selbst, sondern mit einer assoziierten Komponente, dem G-Protein, interagiert. Nach Korner et al. werden spezifische Thiolgruppen des G-Proteins exponiert, wenn das G-Protein mit dem hormonaktivierten Rezeptor interagiert. Demnach ist das Verfahren von Korner et al. nicht geeignet, den eigentlichen Rezeptor zu markieren.

André et al. (Biochemical Pharmacology, Vol. 31, No. 22, 3657-3662, 1982) beschreiben den Effekt von NEM auf agonisten-induzierte Konformationsänderungen des β-adrenergen Rezeptors. Das an Membranpräparationen durchgeführte Verfahren ist durch drei Inkubationsschritte gekennzeichnet: Inkubation mit NEM, gefolgt von einer Inkubation von NEM in Gegenwart des Agonisten (-)-Iso-proterenol und anschließender Inkubation mit dem markierten Antagonisten (-) - [³H]DHA. André et al. beobachteten, daß in ihren Experimenten die durch NEM hervorgerufenen Effekte auch durch GTP induziert werden können, so daß hier offenbar G-Proteine entscheidend beteiligt sind.

Lipson et al. (Biochemistry 1986, 25, 5678-5685) beschreiben ein Verfahren zur Untersuchung des Glucagon-Rezeptor-N-Protein-Komplexes unter Verwendung von N-Ethylmaleimid und anderer, Thiole alkylierender Reagenzien. Es konnte hier z. B. gezeigt werden, daß die Anwesenheit von NEM vor, während oder nach der Assoziationsreaktion von ¹²⁵I-Glucagon und partiell gereinigten, proteinhaltigen Lebermembranen die Freisetzung von gebundenem Hormon, während einer anschließenden Dissoziationsreaktion, begünstigt.

In einem Übersichtsartikel, in dem die Verwendung des alkylierenden Reagenzes N-Ethylmaleimid (NEM) bei der Erforschung G-Protein gekoppelter Rezeptoren zuletzt erwähnt wurde (Lefkowitz et al., Ann. Rev. Biochem. 52, 159 - 86, 1983), merken die Autoren an, daß Unsicherheit darüber besteht, ob die kritischen Sulfhydrylgruppen auf dem Rezeptor, G-Protein oder gar auf beiden Komponenten lokalisiert sind.

Heutzutage ist die besondere Bedeutung der Cysteine im αβ-Heterotrimer des G-Proteins allgemein bekannt. Entsprechende Untersuchungen wurden z. B. von Garcia-Higuera (J. Biol. Chem., Vol. 257, No. 1, 528 - 535, 1996) unter Verwendung von vernetzenden Reagenzien sowie mittels SDM (Site-directed mutagenesis) durchgeführt.

Li et al. (The Journal of Biological Chemistry, Vol. 267, No. 11, 7570 - 7575, 1992) untersuchten die Bedeutung von Thiolgruppen für die Bindung des Neuropeptides Y an den Y₂ Rezeptor.

Untersuchungen hinsichtlich der Bedeutung von Disulfidbrücken und Thiolgruppen für die Bindung des Thyrotropin-Releasing Hormons (TRH) an den TRH-Rezeptor wurden von Cook et al. (Endocrinology, Vol. 137, No. 7, 2851 - 2858, 1996) durchgeführt. Dithiothreitol (DTT), ein Disulfidbrücken-reduzierendes Reagenz, und p-CMB, eine Thiolgruppen-blockierende Substanz, reduzieren in dosisabhängiger Weise die spezifische TRH-Bindung.

Ansätze zur Untersuchung von Konformationsänderungen von G-Protein-gekoppelten Rezeptoren sind oftmals indirekter Natur, d. h. es wird z. B. der Effekt der Rezeptorkonformation auf die GTPase-Aktivität des G-Proteins oder die Aktivität von Effektorenzymen untersucht.

Gether et al. (The Journal of Biological Chemistry, Vol. 270, No. 47, Issue of November 24, pp. 28268 - 28275, 1995) beschreiben jedoch ein Verfahren zur Fluoreszenzmarkierung des gereinigten β₂-adrenergen Rezeptors mit dem Ziel, ligandeninduzierte Konformationsänderungen dieses G-Protein-gekoppelten Rezeptors direkt erkennbar zu machen. Hierzu wurde der in SF-9 Insektenzellen exprimierte Rezeptor zunächst einer Reinigung unterzogen, bevor er mit dem Cysteinspezifischen Fluoreszenzmarker N,N'-dimethyl-N-(iodoacetyl)-N'-(7-nitrobenz-2-oxa-1,3-diazol-4-yl)ethylendiamin (IANBD) markiert wurde. Die Fluoreszenz von IANBD ist stark von der Polarität der Farbstoffumgebung abhängig und kann daher als Indikator für Konformationsänderungen des Rezeptors herangezogen werden. So konnte durch Bindung des Agonisten Isoproterenol an den β₂-adrenergen Rezeptor eine geringe Abnahme der Fluoreszenzemission beobachtet werden. Nachteilig an dem von Gether et al. beschriebenen Verfahren ist insbesondere die äußerst arbeits- und zeitintensive mehrstufige Reinigung des Rezeptors.

Übersichtsartikel über die Superfamilie der nukleären Rezeptoren sind gerade in jüngster Zeit vielfältig erschienen (Cell, Vol. 83, 835 - 839, 841 - 850, 851 - 857, 1995), so daß im Rahmen der vorliegenden Anmeldung nicht näher auf die Funktionsweise dieser Rezeptoren eingegangen werden soll.

Aufgabe der vorliegenden Erfindung ist es, ein Verfahren zur spezifischen Markierung proteinhaltiger Proben wie Rezeptoren - insbesondere Transmembranrezeptoren, aber auch löslichen Rezeptoren - bereit zu stellen.

Die der Erfindung zugrundeliegende Aufgabe wird gelöst durch ein Verfahren gemäß Anspruch 1. Vorteilhafte Weiterbildungen der Erfindung sind in den Unteransprüchen gekennzeichnet.

Das erfindungsgemäße Verfahren ist durch die folgenden Verfahrensschritte gekennzeichnet:
- mindestens einer Inkubation einer proteinhaltigen Probe mit mindestens einem für Selenocyst(e)in- und/oder Cyst(e)ingruppen spezifischen Modifizierungsreagenz, gefolgt von
- mindestens einer weiteren Inkubation der proteinhaitigen Probe mit mindestens einem für Selenocyst (e) in- und/oder Cyst(e)ingruppen spezifischen Markierungsreagenz,
- wobei vor und/oder während und/oder nach mindestens einer der genannten Inkubationen die Zugabe mindestens einer Substanz erfolgt, die mit dem Protein in Wechselwirkung tritt.

Die genannten Gruppen können im Protein im wesentlichen entweder in reduzierter Form (Selenocysteine bzw. Cysteine) oder in oxidierter Form vorliegen (Selenocystine bzw. Cystine). Die Schreibweisen "Selenocyst(e)ine" und "Cyst(e)ine" kennzeichnen, daß die genannten Gruppen im wesentlichen in einem der genannten Redoxzustände vorliegen können.

Das erfindungsgemäße Verfahren nutzt aus, daß durch eine mittels Zugabe einer Substanz induzierte Aktivierung bzw. Konformationsänderung des Proteins anscheinend unzugängliche Selenocyst (e) ine und/oder Cyst(e)ine des Proteins im Rahmen einer Inkubation mit einem für Selenocyst(e)in- und/oder Cyst(e)ingruppen spezifischen Markierungsreagenz markiert werden können bzw. sich der Markierungsgrad des Proteins und/oder Eigenschaften des Markierungsreagenz ändern.

Zum Nachweis einer derartig induzierten Aktivierung bzw. Konformationsänderung ist es wünschenswert, insbesondere bereits vor der Zugabe der mit dem Protein in Wechselwirkung tretenden Substanz zugängliche Selenocyst(e)ine und/oder Cyst(e)ine zu modifizieren, um den Hintergrund zu reduzieren bzw. die Detektionseffizienz zu erhöhen. Dies erfolgt durch den erfindungsgemäßen ein- oder mehrmaligen Zusatz eines für Selenocyst (e) in- und/oder Cyst(e)ingruppen spezifischen Modifizierungsreagenzes. Bei Anwendung des erfindungsgemäßen Verfahrens mit 7-Transmembranrezeptoren können so in vorteilhafter Weise negative allosterische Effekte des G-Proteins auf den Rezeptor, welche die Agonistendissoziation beschleunigen, unterdrückt oder störende Cystein-Proteasen, welche einen Abbau des Agonisten-Rezeptor-Komplexes hervorrufen können, inaktiviert werden. Die für Selenocyst(e)inund/oder Cyst(e)ingruppen spezifischen Modifizierungs- und Markierungs-reagenzien können stöchiometrisch oder gemäß einer weiteren bevorzugten Ausführungsform des erfindungsgemäßen Verfahrens im Überschuß zugesetzt werden. In vorteilhafter Weise kann überschüssiges Modifizierungsreagenz vor der Inkubation der proteinhaltigen Probe mit einem Markierungsreagenz insbesondere durch Zentrifugation oder durch Waschschritte entfernt bzw. durch Zusatz von Cystein, Selenocystein oder SH-Gruppen enthaltenden Chemikalien inaktiviert werden.

Das erfindungsgemäße Verfahren zeichnet sich in vorteilhafter Weise im Vergleich zum Stand der Technik dadurch aus, daß es durch den Einsatz eines einzelnen Markierungsreagenzes für die Anwendung im Bereich des Hochdurchsatz-Screenings besonders geeignet ist und die Detektion neuer Liganden zu insbesondere bekannten proteinhaltigen Proben oder umgekehrt ermöglicht. Eine beschleunigte funktionale Validierung von Orphan-Rezeptoren, insbesondere 7-Transmembran-Orphan-Rezeptoren als Target/Lead-Systeme und bereits bekannter Rezeptoren im Pharma-Screening auf neue Agonisten oder Antagonisten durch ein allgemeines Testverfahren ist von größter wirtschaftlicher Bedeutung. So können beispielsweise mittels des erfindungsgemäßen Verfahrens Agonisten für einen Orphan-Rezeptor mit unbekannter Funktion aus einer Liganden-Library identifiziert werden. Das erfindungsgemäße Verfahren erlaubt die Unterscheidung von Agonisten und Antagonisten. So kann eine Detektion von Antagonisten aufgrund der Inhibierung der agonisten-induzierten Markierung bzw. eine Detektion einer Substrat/Inhibitor-wechselwirkung erfolgen.

Das erfindungsgemäße Verfahren kann eingesetzt werden, um im Rahmen eines Screeningverfahrens Substanzen zu bestimmen, die mit einem zu untersuchenden Protein in Wechselwirkung treten. Dabei können diese Substanzen z. B. aus Naturstoffextrakten stammen. Derzeit enthalten kommerzielle pflanzliche Arzneimittel meist Rohextrakte, chromatographische Fraktionen, Mischungen oder Emulsionen von Substanzen. Es wird allgemein davon ausgegangen, daß zumindest zu einem Viertel die heute in Industrieländern eingesetzten Arzneimittel pflanzlicher Herkunft sind oder nach dem Vorbild pflanzlicher Inhaltsstoffe hergestellt werden. Die Substanzen können jedoch auch dem von der kombinatorischen Chemie zur Verfügung gestellten Substanzreservoir entstammen. Mit ihr lassen sich in kürzester Zeit mehrere Millionen strukturell verwandter Substanzen erzeugen. Hierbei handelt es sich oftmals schon um ausgewählte Verbindungen, bei denen aufgrund der Eigenschaften der Ausgangs-materialien zu erwarten ist, daß zumindest einige in mehr oder weniger ausgeprägter Form die gewünschte Aktivität zeigen. Durch Screening des resultierenden Verbindungspools mittels des erfindungsgemäßen Verfahrens kann man die wirksamsten Verbindungen auslesen.

Unter Anwendung des erfindungsgemäßen Verfahrens ist es auch möglich, im Rahmen eines Screenings insbesondere noch nicht näher charakterisierte Proteine auf eine potentielle Wechselwirkung mit insbesondere bekannten Substanzen zu testen.

Arzneistoffe greifen bevorzugt an Enzymen, Rezeptoren, Transportern, Ionenkanälen und Signalproteinen an. Manche hemmen die durch Enzyme katalysierten Reaktionen, andere lagern sich an Rezeptoren an und erzeugen dadurch entweder als Agonisten dieselbe Wirkung wie die körpereigenen Botenstoffe oder haben als Antagonisten genau den umgekehrten Effekt, indem sie normalen Liganden den Zugang streitig machen oder die Ausbildung einer dreidimensionalen Struktur des Rezeptors behindern. Desgleichen werden bei Transportern die eigentlich zu befördernden Substanzen verdrängt. Im Falle von Ionenkanälen stabilisiert das Pharmakon entweder die offene oder die geschlossene Form. Signalproteine schließlich steuern die Aktivitäten von Enzymen, Rezeptoren oder Ionenkanälen und lassen sich ebenfalls durch Arzneimittel beeinflussen. In allen diesen Fällen ist entscheidend, daß der Arzneistoff mit den biologischen Strukturen kommuniziert oder die Wechselwirkung mit dem eigentlich vorgesehenen Agens blockiert.

Das erfindungsgemäße Verfahren bietet die Möglichkeit, Substanzen und Proteine gleichermaßen dahingehend zu testen, ob sie miteinander in Wechselwirkung treten.

Es kann wünschenswert sein, die Inkubationszeiten an die Art der für Selenocyst(e)in- und/oder Cyst(e)ingruppen spezifischen Modifizierungs- bzw. Markierungsreagenzien anzupassen. Ferner ist es möglich, nach Inkubation der proteinhaltigen Probe mit den für Selenocystein- und/oder Cysteingruppen spezifischen Modifizierungsreagenzien diese zunächst nur mit der Substanz im Sinne einer Prääquilibrierung zu versetzen und erst zu einem späteren Zeitpunkt das für Selenocyst(e)in- und/oder Cyst(e)ingruppen spezifische Markierungsreagenz zuzusetzen. Der Zusatz des Markierungsreagenzes kann ein- oder mehrmalig erfolgen. Es kann jedoch auch wünschenswert sein, zunächst die mit dem Protein in Wechselwirkung tretende Substanz der Probe zuzusetzen und in sich daran anschließenden Schritten den Zusatz des Modifizierungsreagenzes und des Markierungsreagenzes vorzunehmen. Ebenso können auch die anderen, im Anspruch 1 genannten Zeitpunkte des Zusatzes der mit dem Protein in Wechselwirkung tretenden Substanz vorteilhaft sein.

Gemäß einer weiteren Ausführungsform des erfindungsgemäßen Verfahrens werden insbesondere Transmembran-Rezeptoren, wie z. B. G-Protein gekoppelte 7-Transmembran-Rezeptoren, spezifisch markiert bzw. wird eine ligandeninduzierte Aktivierung und/oder Deaktivierung und/oder Konformationsänderung dieser Rezeptoren nachgewiesen.

Für Cyst(e)ingruppen bzw. Selenocyst(e)ingruppen spezifische Reagenzien sind aus der Literatur (Creighton, "Proteins", Freeman & Co., 1984) bekannt und können entsprechend im erfindungsgemäßen Verfahren Verwendung finden. Hierzu wurden Experimente mit verschiedenen für Selenocyst(e)ine bzw. Cyst(e)ine reaktiven Reagenzien (u. a. Iodacetat, Iodacet-amid, Diamid, monovalente und bivalente Maleimide wie NEM, verschiedene Metallverbindungen, DTT, NaBH₄, Pyridoxalphosphat) durchgeführt. In bevorzugter Weise können alkylierende Reagenzien wie N-Ethylmaleimid (NEM) im erfindungsgemäßen Verfahren verwendet werden. Es kann weiterhin bevorzugt sein, eine Inkubation der proteinhaltigen Probe mit einem reduzierenden Reagenz wie β-Mercaptoethanol oder Dithiothreitol durchzuführen, um somit Cysteine, welche durch Disulfidbrücken miteinander verknüpft sind, zu beeinflussen.

Durch Kombination von alkylierenden und reduzierenden Cyst(e)in-reaktiven Reagenzien ist es möglich, die Reaktionsbedingungen weiter zu optimieren. Reagenzien unterschiedlicher Hydrophobizität und Größe weisen eine unterschiedliche Membranpermeabilität auf, welche im erfindungsgemäßen Verfahren berücksichtigt werden kann.

Die Substitution von Cysteinen durch Selenocysteine in Proteinen ist in der jüngeren Literatur beschrieben (Müller et al., Biochemistry, 33, 3404 - 3412, 1994; Ursini et al., Methods in Enzymology, Vol. 252, 38 - 53, Academic Press, 1995; Björnstedt et al., Methods in Enzymology, Vol. 252, 209 - 219, Academic Press, 1995).

Unter einem für Selenocyst(e)in- bzw. Cyst(e)ingruppen spezifischen Markierungsreagenz werden im Sinne der Erfindung jegliche Reagenzien verstanden, welche mit Selenocyst(e)in oder Cyst(e)in reagieren und einen detektierbaren Anteil enthalten. Der detektierbare Anteil kann je nach Detektionssystem gewählt werden, z. B. Haptene im Rahmen einer immunologischen Detektion, multivalente, bevorzugt bivalente, funktionale Gruppen für eine Cross-Linking-Reaktion. Ferner können auch radioaktiv markierte Substanzen verwendet werden oder ein Nachweis mittels Biotin/Streptavidin bzw. Avidin erfolgen. Es kann bevorzugt sein, wie im Ausführungsbeispiel 1 Biotin zu verwenden, so daß die spezifische Markierung eines Rezeptors mittels Western-Blot unter Verwendung entsprechender Antikörper nachgewiesen werden kann. Das für Selenocyst(e)in- bzw. Cyst(e)in spezifische Markierungsreagenz kann in bevorzugter Weise lumineszent sein. Zur Detektion der erfolgten Markierung und/ oder einer Aktivierung und/oder Deaktivierung und/oder Konformationsänderung des Rezeptors können sodann die Methode der Fluoreszenz-Korrelationsspektroskopie (WO 94/16313) sowie andere konfokale Fluoreszenztechniken, wie in der Offenlegungsschrift WO 96/13744 beschrieben sowie der europäischen Patentanmeldung 96 116 373.0 vorgeschlagen, eingesetzt werden. Die letztgenannte Anmeldung schlägt eine Methode zur Analyse von Proben durch wiederholte Messung der Anzahl von Photonen pro definiertem Zeitintervall von Licht vor, welches von den Partikeln in der Probe emittiert, gestreut und/oder reflektiert wird, und Bestimmung der Verteilung der Anzahl von Photonen in den jeweiligen Zeitintervallen, dadurch gekennzeichnet, daß die Verteilung der molekularen Helligkeiten der Partikel aus der Verteilung der Anzahl von Photonen bestimmt wird. Es kann ebenso ein Verfahren zur Analyse von Proben verwendet werden, welches auf die wiederholte Messung der Länge von Zeitintervallen zwischen Photonen zurückgreift und die Verteilung von Eigenschaften der Partikel, wie z. B. die Verteilung der molekularen Helligkeiten, aus der Verteilung der Länge der Zeitintervalle bestimmt. Es kann auch wünschenswert sein, auf ein Detektionsverfahren zurückzugreifen, welches mindestens zwei Eigenschaften der markierten Partikel aus mindestens zweidimensionalen intermediären statistischen Daten bestimmt. Ein derartiges Verfahren ist in der europäischen Patentanmeldung 97 109 353.9 näher erläutert. Es kann weiterhin bevorzugt sein, auf ein Signalanalyseverfahren zur Detektion der Markierungsreaktion zurückzugreifen, welches in der deutschen Patentanmeldung 196 49 048.0 bzw. der internationalen Patentanmeldung PCT/EP 97/06622 beschrieben ist. Hierbei handelt es sich um ein Verfahren zur Unterscheidung oder Erfassung von Partikeln in einer Probe, in der auch mehrere Partikelklassen befindlich sein können, durch Identifizierung von Signalabschnitten zeitaufgelöster, optischer Rohsignale aus der Probe auf der Basis von Einzelphotonendetektion (Einzelpulsdetektion), wobei
- die Probe mindestens zwei Partikelklassen enthält,
- die Probe von einer Lichtquelle beleuchtet wird,
- die von der Probe ausgehenden, aus mindestens einem Meßvolumenelement V, wobei V ≤ 10⁻¹² l, stammenden optischen Rohsignale mittels mindestens einer Detektionseinheit detektiert werden,
- mindestens ein Partikel während seines Aufenthalts im Meßvolumenelement einen Signalanteil erzeugt,
- durch aktiven und/oder passiven Ein- und Wiederaustritt des Partikels in das oder aus dem Meßvolumenelement ein Signalabschnitt der optischen Rohsignale bestimmt wird,
- die optischen Rohsignale in beliebige Abschnitte zerlegt werden,
- für mindestens einen beliebigen Abschnitt mindestens eine auf den optischen Rohsignalen basierende Statistik erstellt wird, und
- die mindestens eine Statistik oder mindestens eine Kombination mehrerer Statistiken nach dem Vorhandensein von Merkmalen, welche für den Signalanteil mindestens einer Partikelklasse charakteristisch sind, bewertet wird. Ferner kann es bevorzugt sein, die Markierungsreaktion unter Anwendung des in der deutschen Patentanmeldung 197 02 914.0 beschriebenen Verfahrens zum Bestimmen vorgegebener Eigenschaften von Zielpartikeln eines Probenmediums zu detektieren. Auf den Offenbarungsgehalt der vorstehend erwähnten Patentanmeldungen, insbesondere hinsichtlich der dort erläuterten Detektions- bzw. Signalanalyseverfahren und hierfür geeigneten Vorrichtungen, wird hiermit ausdrücklich Bezug genommen.

Die Bereitstellung der proteinhaltigen Probe kann insbesondere unter Verwendung von (rekombinanten) Zellen, Geweben, Vesikeln oder künstlichen Membranen erfolgen. In vorteilhafter Weise erfordert das erfindungsgemäße Verfahren nicht zwingenderweise die Reinigung der beteiligten Komponenten.

Die Figur 1 zeigt den Effekt von NEM auf die Interaktion von Thyreotropin (TSH) mit dem TSH-Rezeptor.

Die Figur 2 zeigt den Effekt von DTT auf die Interaktion von Thyreotropin (TSH) mit dem TSH-Rezeptor.

Die Figur 3 zeigt TSH-Bindungskurven.

Die Figur 4 zeigt die Ergebnisse von Western-Blots hinsichtlich der Detektion des TSH-FLAG-Rezeptors sowie der Biotin-Bindung an den Rezeptor.

Die Figur 5 zeigt die Ergebnisse der Western-Blots nach unterschiedlichen NEM- und TSH-Behandlungen des TSH-Rezeptors.

Die Figur 6 zeigt den Effekt von NEM auf die Interaktion von ¹²⁵I-markiertem Ancagonisten (Iodocyanpindolol, ICYP) mit dem β₂-adrenergen Rezeptor in Anwesenheit des Agonisten (-)-Isoproterenol (100 nM).

Die Figur 7 zeigt den Effekt von DTT auf die Interaktion von ¹²⁵I-markiertem Antagonisten (Iodocyanpindolol, ICYP) mit dem β₂-adrenergen Rezeptor in Anwesenheit des Agonisten (-)-Isoproterenol (100 nM).

Die Figur 8 zeigt den Effekt von NEM bzw. DTT auf den Radioligand-Rezeptorassay unter Verwendung des β₂-adrenergen Rezeptors.

Die Figur 9 zeigt die Ergebnisse von Western-Blots hinsichtlich der Detektion des β₂-adrenergen Rezeptors sowie der Biotin-Bindung an den Rezeptor.

Die Figur 10 zeigt den Effekt von NEM auf die Interaktion von ¹²⁵I-markiertem Endothelin-1 mit dem Endothelin (ET_{A})-Rezeptor.

Die Figur 11 zeigt den Effekt von DTT auf die Interaktion von ¹²⁵I-markiertem Endothelin-1 mit dem Endothelin (ET_{A})-Rezeptor.

Die Figur 12 zeigt den Effekt von NEM bzw. DTT auf den Radioligand-ET_{A}-Rezeptorassay unter Verwendung des ET_{A}-Rezeptors.

Die Figur 13 zeigt die Ergebnisse von Western-Blots hinsichtlich der Detektion des ET_{A}-Rezeptors sowie der Biotin-Bindung an den Rezeptor.

Die Figur 14 zeigt den Effekt von Estradiol auf die NEM-Biotin-Bindung an den humanen Östrogen-Rezeptor α (hERα).

Die Figur 15 zeigt die Anwendung des erfindungsgemäßen Verfahrens bei der Untersuchung des humanen Östrogen-Rezeptors α (hERα).

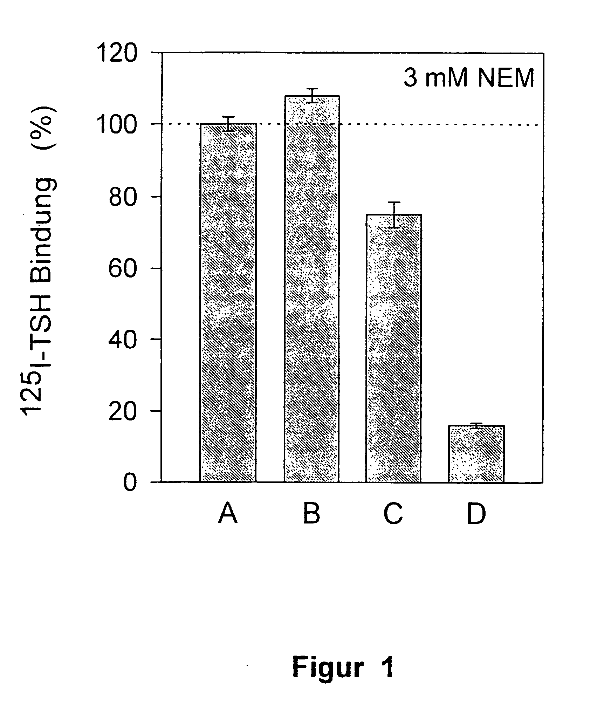

Die Figur 1 verdeutlicht den Effekt von NEM auf die Interaktion von TSH mit dem TSH-Rezeptor. ¹²⁵I-markiertes Rinder-TSH (B) bzw. mit humanem TSH-Rezeptor transfizierte HeLa-Zellen (C) wurden mit 3 mM NEM für 20 min. bei Raumtemperatur inkubiert. Nach Verdünnung bzw. Waschschritten erfolgte der Zusatz von unbehandeltem Rezeptor bzw. Radioligand. Der Assay wurde über Nacht bei 30°C inkubiert und die Rezeptorfraktion anschließend zur Bestimmung des gebundenen ¹²⁵I-TSH präzipitiert. (D) zeigt das Ergebnis eines in Gegenwart von 3 mM NEM durchgeführten Assays, wobei vorher weder Hormon noch Rezeptorfraktion mit NEM vorbehandelt worden sind. (A) zeigt die Kontrollinkubation ohne jegliche NEM-Inkubation.

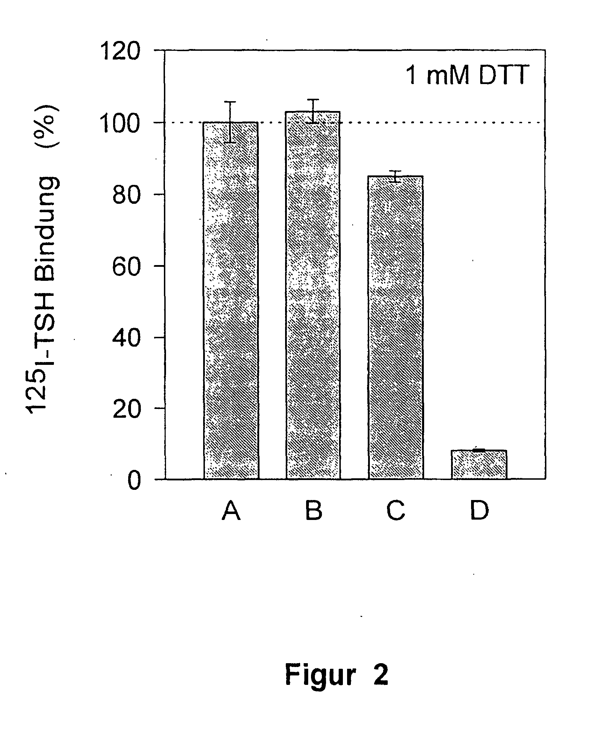

Die Figur 2 verdeutlicht den Effekt von DTT auf die Interaktion von TSH mit dem TSH-Rezeptor. ¹²⁵I-markiertes Rinder-TSH (B) bzw. mit humanem TSH-Rezeptor transfizierte HeLa-Zellen (C) wurden mit 1 mM DTT für 20 min. bei Raumtemperatur inkubiert. Nach Verdünnung bzw. Waschschritten erfolgte der Zusatz von unbehandeltem Rezeptor bzw. Radioligand. Der Assay wurde über Nacht bei 30°C inkubiert und die Rezeptorfraktion anschließend zur Bestimmung des gebundenen ¹²⁵I-TSH präzipitiert. (D) zeigt das Ergebnis eines in Gegenwart von 1 mM DTT durchgeführten Assays, wobei vorher weder Hormon noch Rezeptorfraktion mit DTT vorbehandelt worden sind. (A) zeigt die Kontrollinkubation ohne jegliche DTT-Inkubation.

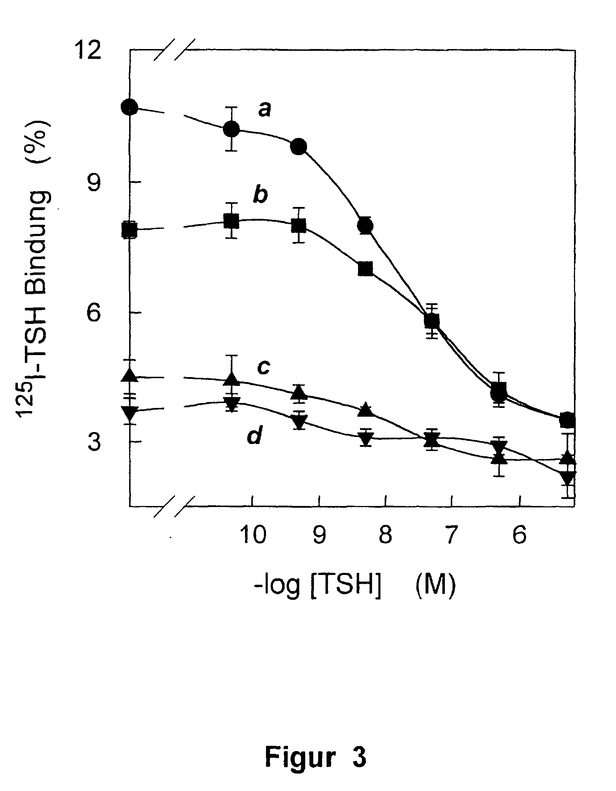

TSH-Bindungskurven mit unbehandeltem Rezeptor als Kontrolle (a), mit 3 mM NEM vorbehandeltem Rezeptor (b), lediglich im anschließenden Bindungsassay mit 3 mM NEM behandeltem Rezeptor (c) sowie Rezeptor, der sowohl mit 3 mM NEM vorbehandelt als auch im Assay mit 3 mM NEM behandelt (d) wurde, sind in Figur 3 dargestellt.

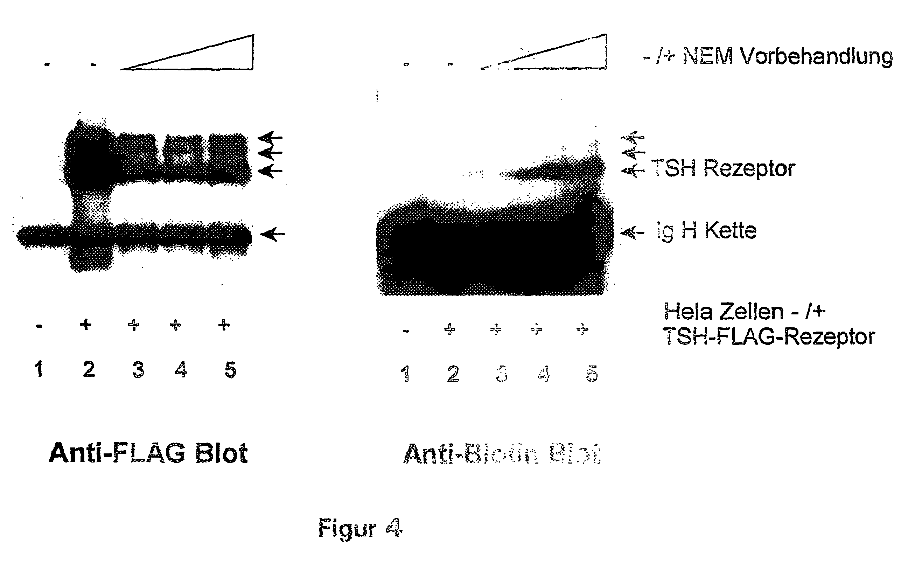

Die Figur 4 zeigt die Ergebnisse der Western-Blots hinsichtlich der Detektion des TSH-FLAG-Rezeptors als Kontrolle (Anti-FLAG-blot) sowie der Detektion des an den TSH-Rezeptor gebundenen cysteinspezifischen Markierungsreagenzes NEM-Biotin. Mit dem TSH-FLAG-Rezeptor transfizierte HeLa-Zellen wurden mit unterschiedlichen NEM-Konzentrationen jeweils einmalig für 30 Minuten inkubiert (Spalte 1: HeLa-Zellen ohne TSH-FLAG-Rezeptor, keine NEM-Vorbehandlung; Spalte 2: HeLa-Zellen mit TSH-FLAG-Rezeptor, keine NEM-Vorbehandlung; Spalte 3: HeLa-Zellen mit TSH-FLAG-Rezeptor, Vorbehandlung mit 100 µM NEM; Spalte 4: HeLa-Zellen mit TSH-FLAG-Rezeptor, Vorbehandlung mit 1 mM NEM, Spalte 5: HeLa-Zellen mit TSH-FLAG-Rezeptor, Vorbehandlung mit 10 mM NEM). Nach Entfernen der NEM-Lösungen sowie Waschen der Zellen wurden die Zellen mit 100 nM TSH und 1 mM NEM-Biotin eine Stunde lang inkubiert. Nach Behandlung mit Zell-Lyse-Puffer wurde der TSH-FLAG-Rezeptor mittels anti-FLAG-Antikörper immunopräzipitiert. Das Immunopräzipitat wurde in zwei Fraktionen aufgeteilt, welche beide mittels SDS-PAGE weiterbehandelt wurden. Die separierten Proteine wurden für die Western-Blots (anti-FLAG bzw. anti-Biotin) auf Nitrocellulose-Membranen transferiert. Die Detektion erfolgte unter Verwendung eines zweiten enzymgekoppelten Antikörpers (anti-Ig). Der TSH-FLAG-Rezeptor zeigt ein charakteristisches Bandenmuster (anti-FLAG blot) bestehend aus einer Hauptbande und zwei Nebenbanden, welches durch die NEM-Behandlung unbeeinflußt blieb. Die Markierung des TSH-Rezeptors mit NEM-Biotin steigt mit zunehmenden Konzentrationen an NEM in der Vorbehandlung (anti-Biotin blot). Höhere NEM-Konzentrationen können entsprechend mehr bereits vorhandene Cystein-SH-Gruppen beeinflussen, so daß weniger NEM-Biotin aufgrund unspezifischer Bindungsreaktionen an den Zellen verloren geht. Als Folge steht relativ mehr NEM-Biotin zur Markierung der durch ligandeninduzierte Aktivierung und/oder Konformationsänderung des Rezeptors zugänglich gemachten SH-Gruppen zur Verfügung.

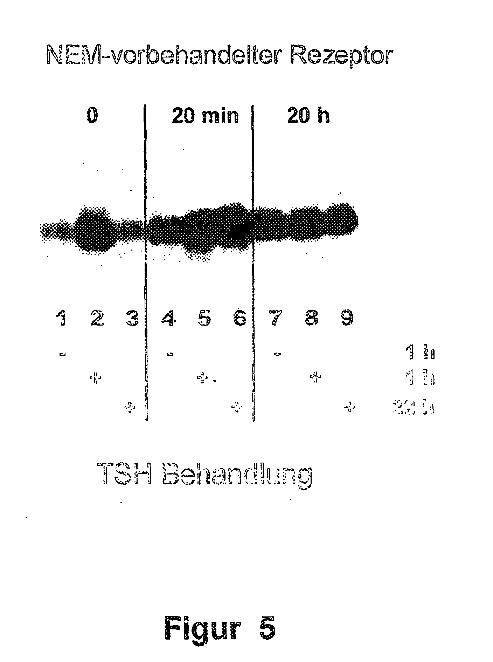

Die Figur 5 zeigt die Ergebnisse der Western-Blots nach unterschiedlichen NEM- und TSH-Behandlungen des TSH-Rezeptors. Die Zellen wurden in drei Fraktionen aufgeteilt und vor der Inkubation mit Puffer (Spalten 1 bis 3) bzw. mit 3 mM NEM (Spalten 4 bis 6: 20-minütige Inkubation; Spalten 7 bis 9: 20-stündige Inkubation) pelletiert. Jede Probe wurde sodann wie folgt weiterbehandelt: einstündige Inkubation mit Puffer (Spalten 1, 4 und 7), einstündige Inkubation mit 100 nM TSH (Spalten 2, 5 und 8) bzw. 22-stündige Inkubation mit 100 nM TSH (Spalten 3, 6 und 9). Es folgte jeweils eine einstündige Inkubation mit 1 mM NEM-Biotin. Die Proben wurden zentrifugiert, mehrmals gewaschen, lysiert und nach SDS-PAGE mittels Western-Blot untersucht. Der TSH-Rezeptor wurde mit anti-FLAG Antikörper immunopräzipitiert und im Blot auf Biotinbindung hin untersucht. Die Spalten 1 und 2 zeigen eine schwache NEM-Biotin-Markierung des Rezeptors, welche sich durch einstündige TSH-Inkubation erhöhen läßt (Spalte 2). Die Spalten 4 bis 6 zeigen zum einen die durch 20-minütige Inkubation mit NEM hervorgerufene erhöhte Markierung des Rezeptors und belegen zum anderen die mittels TSH-Inkubation erzielte höhere Markierungsrate des Rezeptors. Die Spezifität der TSH-induzierten NEM-Biotinmarkierung des Rezeptors geht offenbar durch lange NEM-Inkubationszeiten verloren (Spalten 7 bis 9).

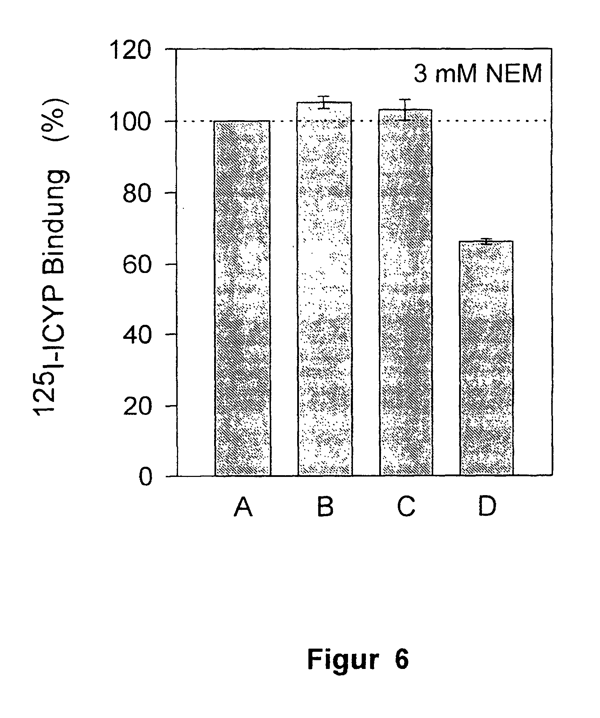

Die Figur 6 verdeutlicht den Effekt von NEM auf die Interaktion von Ligand mit dem β₂-adrenergen Rezeptor. Agonist (-)-Isoproterenol (B) bzw. den humanen β₂-adrenergen Rezeptor exprimierende A431-Zellen (C) wurden mit 3 mM NEM für 20 min. bei Raumtemperatur inkubiert. Nach Verdünnung bzw. Waschschritten erfolgte der Zusatz von unbehandeltem Rezeptor bzw. Ligand in Anwesenheit von ¹²⁵I-ICYP. Der Assay wurde 40 min. bei 30°C inkubiert und die Rezeptorfraktion anschließend zur Bestimmung des gebundenen ¹²⁵I-ICYP präzipitiert. Die Vorbehandlung des Agonisten oder des Rezeptors mit NEM hat nur einen geringen Effekt auf die Bindung. (D) zeigt das Ergebnis eines in Gegenwart von 3 nM NEM durchgeführten Assays, wobei vorher weder Hormon noch Rezeptorfraktion mit NEM vorbehandelt worden sind. (A) zeigt die Kontrollinkubation ohne jegliche NEM-Inkubation.

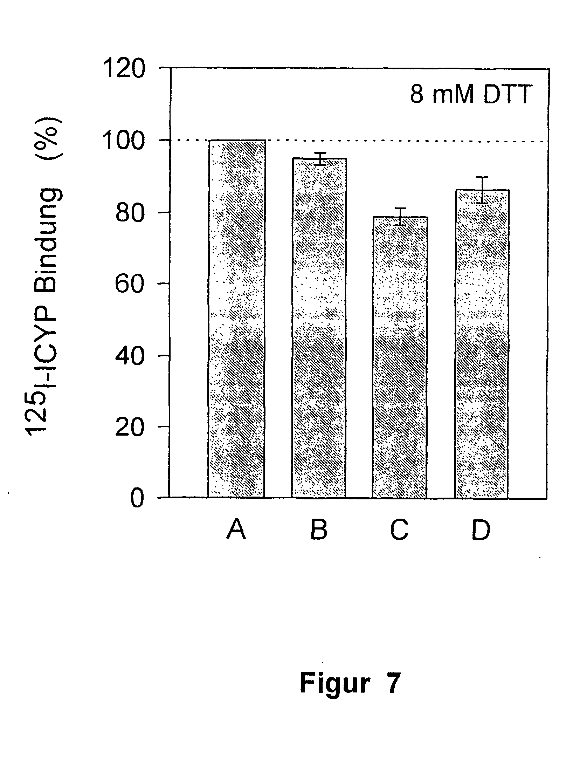

Die Figur 7 verdeutlicht den nur geringen Effekt von DTT auf die Interaktion von Ligand mit dem β₂-adrenergen Rezeptor. Agonist (-)-Isoproterenol (B) bzw. den humanen β₂-adrenergen Rezeptor exprimierende A431-Zellen (C) wurden mit 8 mM DTT für 20 min. bei Raumtemperatur inkubiert. Nach Verdünnung bzw. Waschschritten erfolgte der Zusatz von unbehandeltem Rezeptor bzw. Ligand in Anwesenheit von ¹²⁵I-ICYP. Der Assay wurde 40 min. bei 30°C inkubiert und die Rezeptorfraktion anschließend zur Bestimmung des gebundenen ¹²⁵I-ICYP präzipitiert. (D) zeigt das Ergebnis eines in Gegenwart von 8 mM DTT durchgeführten Assays, wobei vorher weder Hormon noch Rezeptorfraktion mit DTT vorbehandelt worden sind. (A) zeigt die Kontrollinkubation ohne jegliche DTT-Inkubation.

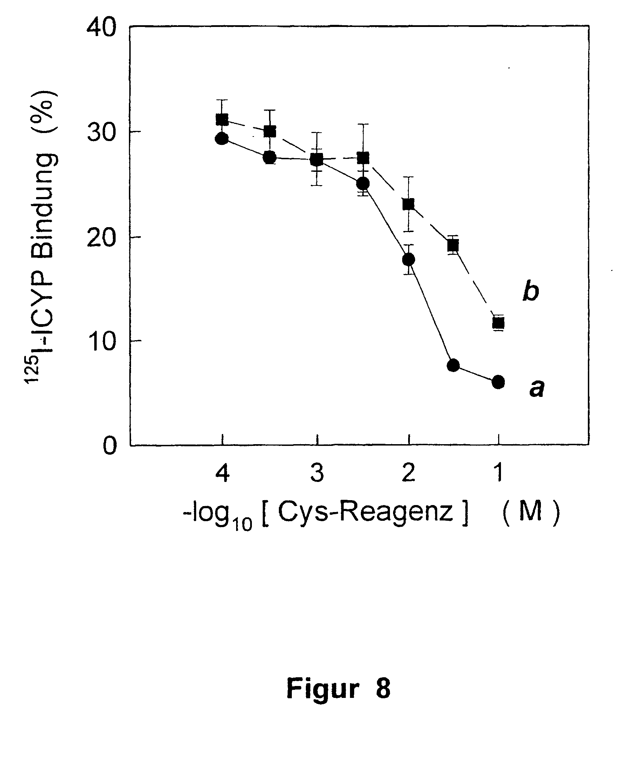

¹²⁵I-ICYP-Bindungskurven mit Rezeptor in Anwesenheit von verschiedenen Mengen NEM (a) bzw. DTT (b) sind in Figur 8 dargestellt.

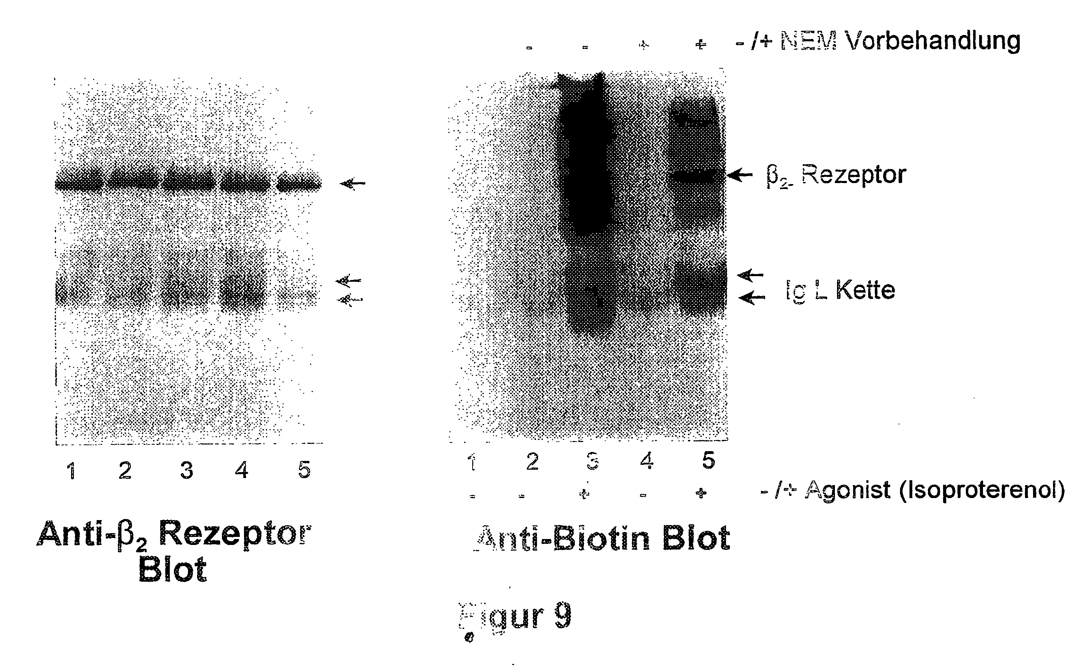

Die Figur 9 zeigt die Ergebnisse der Western-Blots hinsichtlich der Detektion des β₂-adrenergen Rezeptors als Kontrolle (anti-β₂-adrenerger Rezeptor blot) sowie der Detektion des an den β₂-adrenergen Rezeptor gebundenen, für Cysteine spezifischen Markierungsreagenzes NEM-Biotin. A431-Zellen wurden mit (Spalten 4 und 5) bzw. ohne (Spalten 1, 2 und 3) NEM (10 mM) für 10 min. inkubiert. Nach Entfernen der NEM-Lösungen sowie Waschen der Zellen wurden die Zellen mit 100 µM (-)-Isoproterenol für 5 min. inkubiert (Spalten 3 und 5). Es folgte eine einstündige Inkubation mit 1 mM NEM-Biotin (Spalten 2 bis 5), wobei in den Spalten 3 und 5 (-)-Isoproterenol noch anwesend war. Nach Behandlung mit RIPA-Puffer wurde der β₂-adrenerge Rezeptor mittels anti-β₂-adrenerger Rezeptor-Antikörper immunopräzipitiert. Das Immunopräzipitat wurde in zwei Fraktionen aufgeteilt, welche beide mittels SDS-PAGE weiterbehandelt wurden. Die separierten Proteine wurden für die Western-Blots (anti-β-adrenerger Rezeptor blot bzw. anti-Biotin blot) auf PVDF-Membranen transferiert. Die Detektion erfolgte unter Verwendung eines zweiten enzymgekoppelten Antikörpers (anti-Ig). Der β₂-adrenerge Rezeptor zeigt ein charakteristisches Banden-muster (anti-β₂adrenerger Rezeptor blot) bestehend aus einer spezifischen Bande, welches durch die NEM-Behandlung unbeeinflußt blieb. Die Markierung des Rezeptors mit NEM-Biotin ist nur bei Zugabe von Agonist nachweisbar. Durch ligandeninduzierte Aktivierung bzw. Konformationsänderung des Rezeptors zugänglich gemachte SH-Gruppen werden offenbar nunmehr durch NEM-Biotin markiert. Es zeigt sich im Vergleich der Spalten 3 und 5, daß die NEM-Vorbehandlung in vorteilhafter Weise die Spezifität der Nachweisreaktion erhöht.

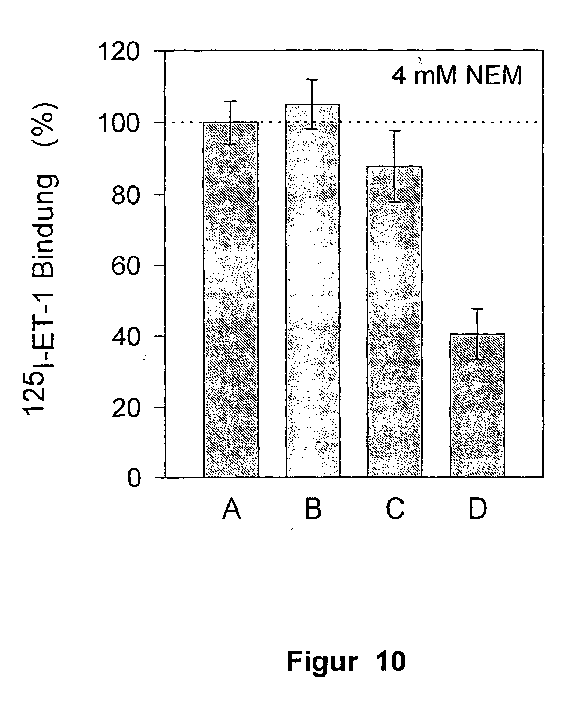

Die Figur 10 verdeutlicht den Effekt von NEM auf die Interaktion von Ligand mit dem Endothelin-Rezeptor. ¹²⁵I-markiertes Endothelin-1 (B) bzw. humaner Endothelin-Rezeptor (C) wurden mit 4 mM NEM für 20 min. bei Raumtemperatur inkubiert. Nach Verdünnung bzw. Waschschritten erfolgte der Zusatz von unbehandeltem Rezeptor bzw. Radioligand. Der Assay wurde über Nacht bei 30°C inkubiert und die Rezeptorfraktion anschließend zur Bestimmung des gebundenen ¹²⁵I-Endothelin präzipitiert. Die Vorbehandlung des Agonisten oder des Rezeptors mit NEM hat nur einen geringen Effekt auf die Bindung. (D) zeigt das Ergebnis eines in Gegenwart von 4 mM NEM durchgeführten Assays, wobei vorher weder Hormon noch Rezeptorfraktion mit NEM vorbehandelt worden sind. (A) zeigt die Kontrollinkubation ohne jegliche NEM-Inkubation.

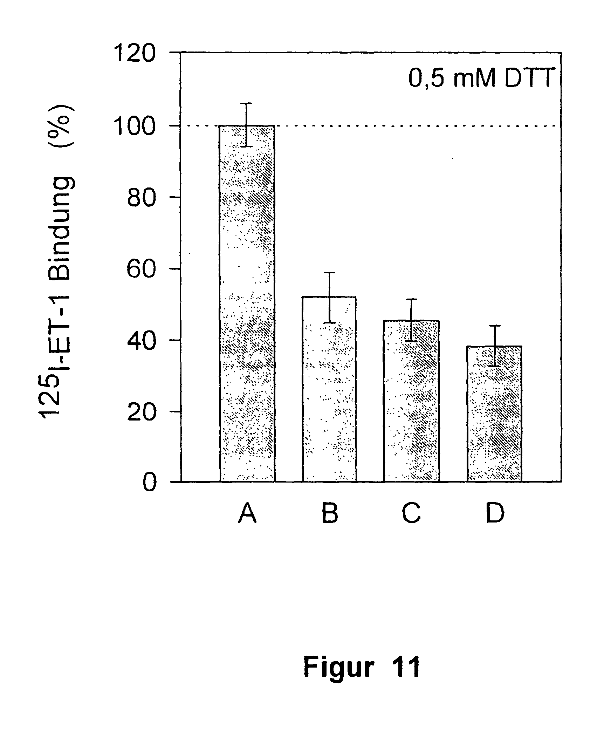

Die Figur 11 verdeutlicht den Effekt von DTT auf die Interaktion von Ligand mit dem Endothelin-Rezeptor. ¹²⁵I-markiertes Endothelin-1 (B) bzw. humaner Endothelin-Rezeptor (C) wurden mit 0,5 mM DTT für 20 min. bei Raumtemperatur inkubiert. Nach Verdünnung bzw. Waschschritten erfolgte der Zusatz von unbehandeltem Rezeptor bzw. Radioligand. Der Assay wurde über Nacht bei 30°C inkubiert und die Rezeptorfraktion anschließend zur Bestimmung des gebundenen ¹²⁵I-Endothelin präzipitiert. Die Vorbehandlung des Agonisten bzw. des Rezeptors mit DTT wirkt sich auf die Bindung aus. (D) zeigt das Ergebnis eines in Gegenwart von 0,5 mM DTT durchgeführten Assays, wobei weder Hormon noch Rezeptorfraktion mit DTT vorbehandelt worden sind. (A) zeigt die Kontrollinkubation ohne jegliche DTT-Inkubation.

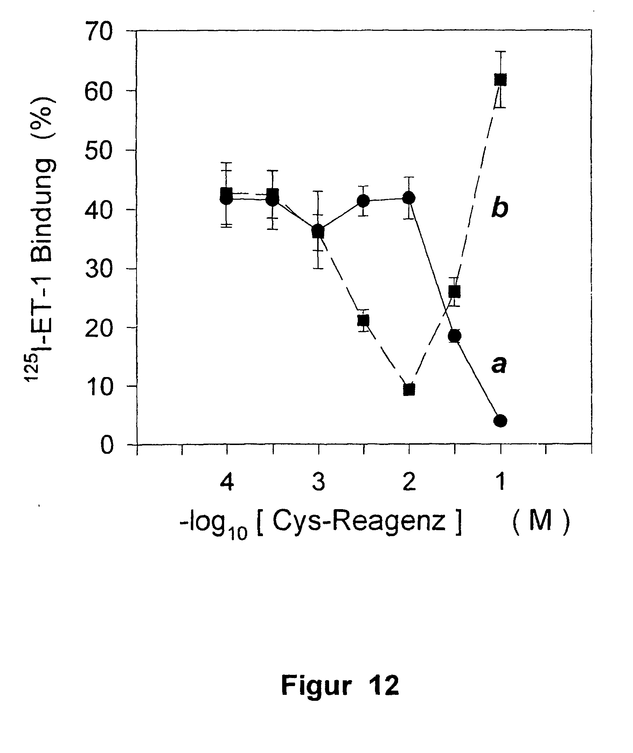

¹²⁵I-Endothelin-Bindungskurven mit Rezeptor in Anwesenheit von verschiedenen Mengen NEM (a) bzw. DTT (b) sind in Figur 12 dargestellt. Offenbar werden die Disulfidbrücken des ¹²⁵I-Endothelins bei hohen DTT-Konzentrationen aufgebrochen.

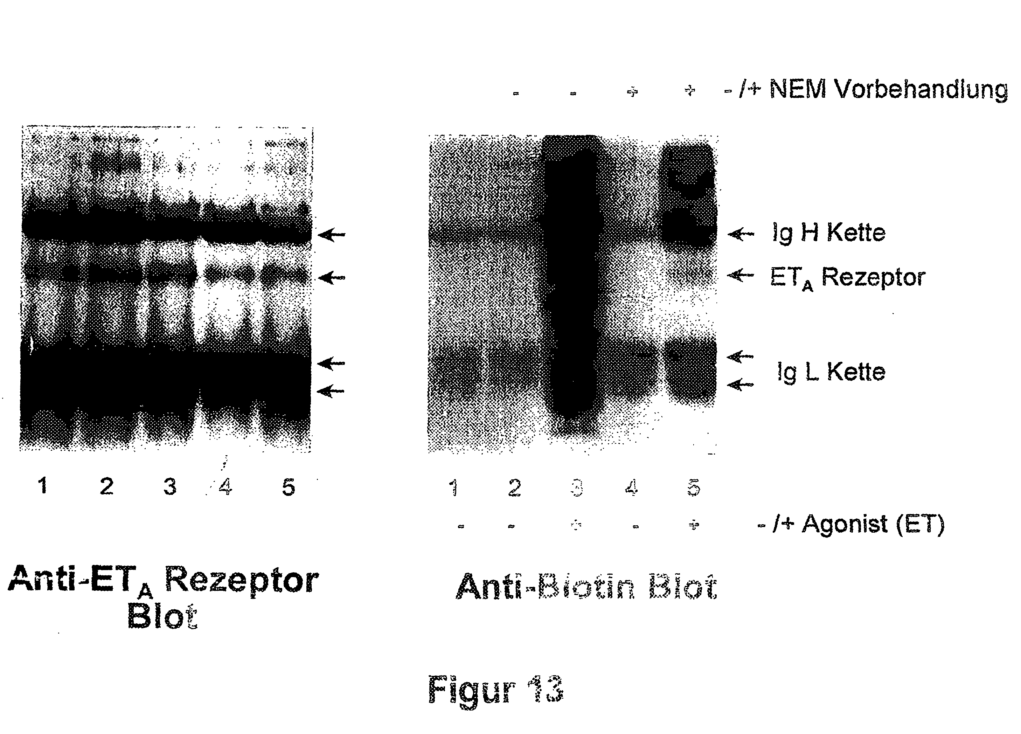

Die Figur 13 zeigt die Ergebnisse der Western-Blots hinsichtlich der Detektion des Endothelin-Rezeptors als Kontrolle (anti-Endothelin-Rezeptor-Blot) sowie der Detektion des an den Endothelin-Rezeptor gebundenen, für Cysteine spezifischen Markierungsreagenzes NEM-Biotin. RAT2-Zellen wurden mit (Spalten 4 und 5) bzw. ohne (Spalten 1, 2 und 3) NEM (10 mM) für 10 min. inkubiert. Nach Entfernen der NEM-Lösungen sowie Waschen der Zellen wurden die Zellen mit 3 nM Endothelin für 10 min. (Spalten 3 und 5) inkubiert. Es folgte eine zweistündige Inkubation mit 1 mM NEM-Biotin (Spalten 2 - 5), wobei in Spalten 3 und 5 Endothelin noch anwesend war. Nach Behandlung mit RIPA-Puffer wurde der Endothelin-Rezeptor mittels anti-Endothelin-Rezeptor-Antikörper immunopräzipitiert. Das Immunopräzipitat wurde in zwei Fraktionen aufgeteilt, welche beide mittels SDS-PAGE weiterbehandelt wurden. Die separierten Proteine wurden für die Western-Blots (anti-Endothelin-Rezeptor bzw. anti-Biotin) auf PVDF-Membranen transferiert. Die Detektion erfolgte unter Verwendung eines zweiten enzymgekoppelten Antikörpers (anti-Ig). Der Endothelin-Rezeptor zeigt ein charakteristisches Bandenmuster (anti-Endothelin-Rezeptor blot) bestehend aus einer spezifischen Bande, welches durch die NEM-Behandlung unbeeinflußt blieb. Die Markierung des Endothelin-Rezeptors mit NEM-Biotin ist nur bei Zugabe von Agonist nachweisbar. Durch ligandeninduzierte Aktivierung bzw. Konformationsänderung des Rezeptors zugänglich gemachte SH-Gruppen werden offenbar nunmehr durch NEM-Biotin markiert. Auch hier zeigt sich, daß die NEM-Vorbehandlung in vorteilhafter Weise die Spezifität der Nachweisreaktion erhöht (Spalten 3 und 5).

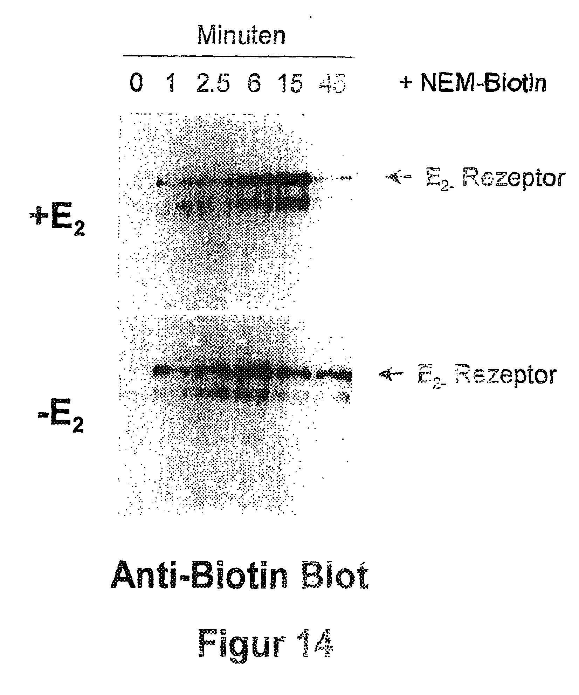

Figur 14 zeigt den Effekt von Estradiol auf die Bindung von NEM-Biotin an den humanen Östrogen-Rezeptor. Nähere Erläuterungen sind im Beispiel 4 gegeben.

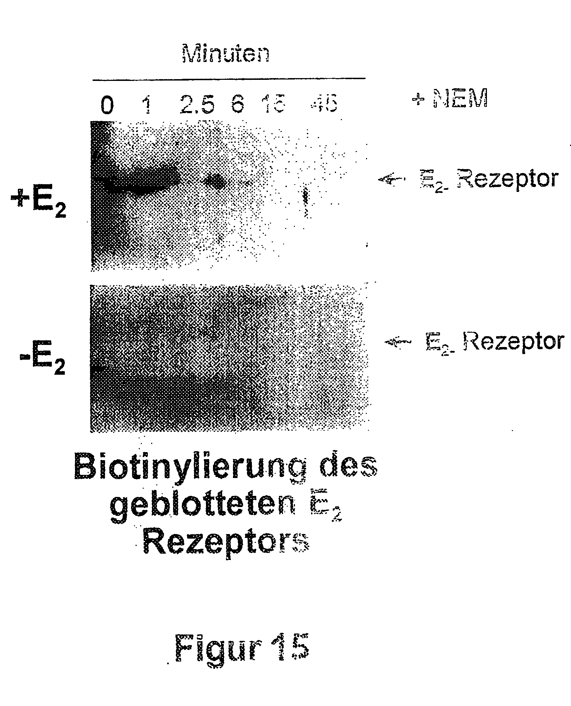

Figur 15 zeigt den Effekt von Estradiol auf die Zugänglichkeit von Cyst(e)inen im Östrogenrezeptor hERα. Das erfindungsgemäße Verfahren wurde hier in folgender Reihenfolge durchgeführt: Zusatz von Ligand (Estradiol), Zusatz von Modifizierungsreagenz (NEM), Zusatz von Markierungsreagenz (NEM-Biotin). Nähere Erläuterungen sind im Beispiel 4 gegeben.

### Beispiel 1:

### Spezifische Markierung und/oder Bestimmung von Konformationsänderungen des TSH-Rezeptors durch Verwendung von biotinyliertem NEM als Cystein-reaktivem Markierungsreagenz (vgl. Figuren 1 bis 5)

Zellen, welche den mit einem FLAG-Epitop versehenen TSH-Rezeptor enthielten, wurden in Assaypuffer (10 mM Kaliumphosphat mit 0,1 g/l BSA, 0,01 g/l Natriumazid und 0,01 g/l Phenolrot, pH 7,4) mit 3 mM NEM für 30 min. bei Raumtemperatur inkubiert. Nachfolgend wurde der Überschuß an Reagenz durch Zentrifugation (1.000 g, 5 min.) entfernt. Es folgte sodann eine Inkubation über Nacht mit 100 µM TSH in Gegenwart von 300 µM biotinyliertem NEM. Die Reaktionseffizienz von biotinyliertem NEM wurde zuvor in einem gesonderten Assay ermittelt und entsprach der von NEM. Sodann wurde die Rezeptorfraktion zweimal zentrifugiert (2 x 10.000 g, 2 min.), die Überstände wurden entfernt und die Zellen mit 1 ml kaltem Protein-Lyse-Puffer (25 mM TRIS-HCl, pH 7,6, 150 mM NaCl, 0,1 g/l Nonident P-40 mit 1 Tab. Boehringer "Complete Protease Inhibitor Complex") bedeckt. Nach 20-minütiger Inkubation bei 4°C auf Eis wurden die Zellen abgeschabt und unerwünschte Bestandteile durch Zentrifugation (10.000 g, 2 min.) pelletiert. 250 µl des Zell-Lysates wurden mit 5 µl des Anti-FLAG-Antikörpers versetzt und 60 min bei 4°C inkubiert. Nach Zentrifugation (10.000 g, 15 min., 4°C) und Abnehmen des Überstandes erfolgte die Zugabe von Protein A-Sepharose-Beads. Es schloß sich eine weitere 60-minütige Inkubation bei 4°C an. Anschließend erfolgte eine Zentrifugation (6.000 g, 30 sec.). Es folgten mehrere Waschschritte (20 mM TRIS-HCl, pH 8, 100 mM NaCl, 0,05 g/l Nonident P-40) mit wiederholten Zentrifugationen. Das Pellet wurde in 150 µl Waschpuffer wieder aufgenommen. 40 µl dieser Probe wurden sodann mit 8 µl Probenpuffer (50 mM Tris-HCl, pH 6,8, 4 % SDS, 12 % Glycin, 2 % β-Mercaptoethanol, 0,01 % SERVA Blue G) versetzt. Die Proben wurden sodann für 3 bis 5 min. aufgekocht, kurz zentrifugiert und anschließend mittels diskontinuierlicher 10 %iger SDS-PAGE getrennt. Nach der Elektrophorese wurde das Gel entnommen, in Blotting-Puffer (48 mM Tris, 39 mM Glycin, 20 % Methanol, 0,037 % SDS) gewaschen und auf einer PVDF-Membran geblottet. Die Membran wurde über Nacht bei Raumtemperatur getrocknet und sodann mit 20 % Methanol versetzt. Zur Verminderung des Hintergrundes wurde die Membran für 1 Stunde geblockt (1 % w/v Casein-Hydrolysat). Der Blockingpuffer wurde anschließend entfernt. Die Membran wurde mit 10 ml einer geeigneten Erst-Antikörper-Verdünnung (Anti-Biotin 1 : 500 und für die Kontrolle Anti-FLAG 1 : 1.000, jeweils in 20 mM TRIS-HCl, pH 7,6, 137 mM NaCl, 0,05 % TWEEN 20, 0,1 % Casein-Hydrolysat) für 1 Stunde inkubiert, 3-malig für 10 min. gewaschen (20 mM TRIS-HCl, pH 7,6, 137 mM NaCl, 0,05 % TWEEN 20) und sodann mit dem jeweiligen zweiten Antikörper (1 : 4.000 als Peroxidase-Konjugate, in 20 mM TRIS-HCl, pH 7,6, 137 mM NaCl, 0,05 % TWEEN 20, 0,1% Casein-Hydrolysat) für 1 Stunde inkubiert. Nach mehrmaligem Waschen (20 mM TRIS-HCl, pH 7,6, 137 mM NaCl, 0,05 % TWEEN 20), zuletzt mit Wasser, wurden jeweils 5 ml ECL-Lösung 1 und 2 zugegeben und der Blot darin 60 Sekunden lang getränkt. Anschließend wurde ein Film aufgelegt. Die Expositionszeit richtete sich nach der Signalstärke.

### Beispiel 2:

### Spezifische Markierung und/oder Bestimmung von Konformationsänderungen des β₂-adrenergen Rezeptors durch Verwendung von biotinyliertem NEM als Cystein-reaktivem Markierungsreagenz (vgl. Figuren 6 bis 9)

Den β₂-adrenergen Rezeptor exprimierende A431 Fibroblasten wurden in DMEM (Dulbeccos modified Eagle medium) mit 1 % BSA und 10 mM NEM für 10 min. bei Raumtemperatur inkubiert. Nachfolgend wurde der Überschuß an Reagenz durch Waschen mittels DMEM mit 1 % BSA entfernt. Es folgte sodann eine Inkubation für 1 Stunde mit 100 µM (-)-Isoproteronol in Gegenwart von 1 mM biotinyliertem NEM. Die Reaktionseffizienz von biotinyliertem NEM wurde zuvor in einem gesonderten Assay ermittelt. Sodann wurden die Zellen von der Kulturflasche durch leichtes Kratzen entfernt und anschließend wurde die Rezeptorfraktion zweimal zentrifugiert (2 x 10.000 g, 2 min.), die Überstände wurden entfernt und die Zellen mit 1 ml kaltem RIPA Puffer (0,1 % SDS, 1 % Triton X100, 1 % sodium deoxycholate, 0,15 M NaCl, 0,01 M Tris-HCl pH 7,4, 1mM EDTA, 1 Tab. Boehringer "Complete Protease Inhibitor Complex") bedeckt. Nach 20-minütiger Inkubation bei 4°C auf Eis wurden die Zellen abgeschabt und unerwünschte Bestandteile durch Zentrifugation (10.000 g, 2 min.) pelletiert. 1.000 µl des Zell-Lysates wurden mit 10 µl Normal Rabbit Serum versetzt und 60 min. bei 4°C inkubiert. Nach Zentrifugation (10.000 g, 15 min., 4°C) und Abnehmen des Überstandes erfolgte die Zugabe von Protein A Sepharose-Beads. Es schloß sich eine weitere 60-minütige Inkubation bei 4°C an. Nach Zentrifugation (10.000 g, 15 min., 4°C) und Abnehmen des Überstandes erfolgte die Zugabe von 4 µg des spezifischen anti-β₂-adrenergen Rezeptor-Antikörpers. Es schloß sich eine weitere 60-minütige Inkubation bei 4°C an. Nach Zentrifugation (10.000 g, 15 min., 4°C) und Abnehmen des Überstandes erfolgte die Zugabe von Protein A-Sepharose. Anschließend erfolgte eine Zentrifugation (6.000 g, 30 sec.). Es folgten mehrere Waschschritce (0,1 % SDS, 1% Triton X100, 1 % sodium deoxycholate, 0,15M NaCl, 0,01M Tris-HCl pH 7,4, 1mM EDTA, 1 Tab. Boehringer "Complete Protease Inhibitor Complex") mit wiederholten Zentrifugationen. Das Pellet wurde in 30 µl Probenpuffer (50 mM Tris-HCl, pH 6,8, 4 % SDS, 12 % Glycin, 2 % β-Mercaptoethanol, 0,01 % SERVA Blue G) aufgenommen. Die Proben wurden sodann für 3 bis 5 min. aufgekocht, kurz zentrifugiert und anschließend mittels diskontinuierlicher 10 %-iger SDS-PAGE getrennt. Nach der Elektrophorese wurde das Gel entnommen, in Blotting-Puffer (48 mM Tris, 39 mM Glycin, 20 % Methanol, 0,037 % SDS) gewaschen und auf einer PVDF-Membran geblottet. Die Membran wurde über Nacht bei Raumtemperatur getrocknet und sodann mit 20 % Methanol versetzt. Zur Verminderung des Hintergrundes wurde die Membran für 1 Stunde geblockt (1 % w/v Casein-Hydrolysat). Dec Blockingpuffer wurde anschließend entfernt. Die Membran wurde mit 10 ml einer geeigneten Erst-Antikörper-Verdünnung (Anti-Biotin 1 : 2.500 und für die Kontrolle Anti-β2-adrenerger Rezeptor 1 : 1.000 jeweils in 20 mM TRIS-HCl, pH 7,6, 137 mM NaCl, 0,05 % TWEEN 20, 0,1 % Casein-Hydrolysat) für 1 Stunde inkubiert, 3-malig für 10 min. gewaschen (20 mM TRIS-HCl, pH 7,6, 137 mM NaCl, 0,05 % TWEEN 20) und sodann mit dem jeweiligen zweiten Antikörper (1 : 2.500 als Peroxidase-Konjugate, in 20 mM TRIS-HCl, pH 7,6, 137 mM NaCl, 0,05 % TWEEN 20, 0,1 % Casein-Hydrolysat) für 1 Stunde inkubiert. Nach mehrmaligem Waschen (20 mM TRIS-HCl, pH 7,6, 137 mM NaCl, 0,05 % TWEEN 20), zuletzt mit Wasser, wurden jeweils 5 ml ECL-Lösung 1 und 2 zugegeben und der Blot darin 60 Sekunden lang getränkt. Anschließend wurde ein Film aufgelegt. Die Expositionszeit richtete sich nach der Signalstärke.

### Beispiel 3:

### Spezifische Markierung und/oder Bestimmung von Konformationsänderungen des Endothelin-Rezeptors durch Verwendung von biotinyliertem NEM als Cystein-reaktivem Markierungsreagenz (vgl. Figuren 10 bis 13)

Den Endothelin-Rezeptor exprimierende RAT2 Fibroblasten wurden in DMEM (Dulbeccos modified Eagle medium) mit 1 % BSA und 10 mM NEM für 10 min. bei Raumtemperatur inkubiert. Nachfolgend wurde der Überschuß an Reagenz durch Waschen mittels DMEM mit 1 % BSA entfernt. Es folgte sodann eine Inkubation für 2 Stunden mit 3 nM Endothelin in Gegenwart von 1 mM biotinyliertem NEM. Die Reaktionseffizienz von biotinyliertem NEM wurde zuvor in einem gesonderten Assay ermittelt. Sodann wurden die Zellen von der Kulturflasche durch leichtes Kratzen entfernt und anschließend wurde die Rezeptorfraktion zweimal zentrifugiert (2 x 10.000 g, 2 min.), die Überstände wurden entfernt und die Zellen mit 1 ml kaltem RIPA Puffer (0,1 % SDS, 1 % Triton X100, 1 % sodium deoxycholate, 0,15 M NaCl, 0,01 M Tris-HCl pH 7,4, 1 mM EDTA, 1 Tab. Boehringer "Complete Protease Inhibitor Complex") bedeckt. Nach 20-minütiger Inkubation bei 4°C auf Eis wurden die Zellen abgeschabt und unerwünschte Bestandteile durch Zentrifugation (10.000 g, 2 min.) pelletiert. 1.000 µl des Zell-Lysates wurden entweder mit 10 µl Normal Rabbit Serum oder Normal Sheep Serum versetzt (in Abhängigkeit des verwendeten primären Anti-Rezeptor Antikörpers) und 60 min. bei 4°C inkubiert. Nach Zentrifugation (10.000 g, 15 min., 4°C) und Abnehmen des Überstandes erfolgte die Zugabe von Protein G Sepharose-Beads. Es schloß sich eine weitere 60-minütige Inkubation bei 4°C an. Nach Zentrifugation (10.000 g, 15 min., 4°C) und Abnehmen des Überstandes erfolgte die Zugabe von 4 µg des spezifischen anti-Endothelin-Rezeptor-Antikörpers. Es schloß sich eine weitere 60-minütige Inkubation bei 4°C an. Nach Zentrifugation (10.000 g, 15 min., 4°C) und Abnehmen des Überstandes erfolgte die Zugabe von Protein G-Sepharose. Anschließend erfolgte eine Zentrifugation (6.000 g, 30 sec.). Es folgten mehrere Waschschritte (0,1 % SDS, 1 % Triton X100, 1 % sodium deoxycholate, 0,15 M NaCl, 0,01 M Tris-HCl pH 7,4, 1 mM EDTA, 1 Tab. Boehringer "Complete Protease Inhibitor Complex") mit wiederholten Zentrifugationen. Das Pellet wurde in 30 µl Probenpuffer (50 mM Tris-HCl, pH 6,8, 4 % SDS, 12 % Glycin, 2 % β-Mercaptoethanol, 0,01 % SERVA Blue G) aufgenommen. Die Proben wurden sodann für 3 bis 5 min. aufgekocht, kurz zentrifugiert und anschließend mittels diskontinuierlicher 10 %-iger SDS-PAGE getrennt. Nach der Elektrophorese wurde das Gel entnommen, in Blotting-Puffer (48 mM Tris, 39 mM Glycin, 20 % Methanol, 0,037 % SDS) gewaschen und auf einer PVDF-Membran geblottet. Die Membran wurde über Nacht bei Raumtemperatur getrocknet und sodann mit 20 % Methanol versetzt. Zur Verminderung des Hintergrundes wurde die Membran für 1 Stunde geblockt (1 % w/v Casein-Hydrolysat). Der Blockingpuffer wurde anschließend entfernt. Die Membran wurde mit 10 ml einer geeigneten Erst-Antikörper-Verdünnung (Anti-Biotin 1 : 2.500 und für die Kontrolle Anti-Endothelin-Rezeptor 1 : 1.000 jeweils in 20 mM TRIS-HCl, pH 7,6, 137 mM NaCl, 0,05 % TWEEN 20, 0,1 % Casein-Hydrolysat) für 1 Stunde inkubiert, 3-malig für 10 min. gewaschen (20 mM TRIS-HCl, pH 7,6, 137 mM NaCl, 0,05 % TWEEN 20) und sodann mit dem jeweiligen zweiten Antikörper (1 : 2.500 als Peroxidase-Konjugate, in 20 mM TRIS-HCl, pH 7,6, 137 mM NaCl, 0,05 % TWEEN 20, 0,1 % Casein-Hydrolysat) für 1 Stunde inkubiert. Nach mehrmaligem Waschen (20 mM TRIS-HCl, pH 7,6, 137 mM NaCl, 0,05 % TWEEN 20), zuletzt mit Wasser, wurden jeweils 5 ml ECL-Lösung 1 un 2 zugegeben und der Blot darin 60 Sekunden lang getränkt. Anschließend wurde ein Film aufgelegt. Die Expositionszeit richtete sich nach der Signalstärke.

### Beispiel 4:

### Spezifische Markierung und/oder Bestimmung von Konformationsänderungen des humanen Östrogen-Rezeptors (hERα) durch Verwendung von biotinyliertem NEM als Cystein-reaktivem Markierungsreagenz

Der humane Östrogen-Rezeptor α (hERα) wurde als rekombinantes Protein in Baculovirus-infizierten Sf9-Zellen exprimiert und durch 17β-Estradiol-Agarose-Chromatographie gereinigt. 2 µg des affinitätsgereinigten hERα in 50 mM Tris pH 7,5, 10 % Glycerol, 0,5 M KCl, 1 mM EDTA, 2 mM DTT, 1 mM sodium vanadtae und 0,02 % Sodiumazid wurden verdünnt auf eine Konzentration von 2 ng/µl in 1 ml TEG-Puffer (10 mM Tris, 10 % Glycerol, 1 mM EDTA, 0,5 mM DTT) bei pH 8,0 und in zwei gleiche Fraktionen aufgeteilt. Zu einer Fraktion wurden 50 µl TEG-Puffer ("minus estradiol", - E₂), zu der anderen Fraktion 50 µl des 17β-Estradiols in TEG-Puffer ("plus estradiol", + E₂) zugesetzt, um eine Estradiol-konzentration von 200 µM zu erzielen. Sättigung der Liganden-bindungsdomäne wurde durch 20-minütige Inkubation bei Raumtemperatur erzielt. Danach wurde der Inkubationsansatz heruntergekühlt. Alle nachfolgenden Schritte wurden bei 4°C durchgeführt, um die Dissoziationsrate des Hormon-Rezeptor-Komplexes auf ein niedriges Level zu reduzieren. Die "minus" und "plus" Estradiol-Aliquots wurden jeweils in weitere 2 Fraktionen aufgeteilt. Zum einen "minus" - "plus"-Paar wurden 10 µl einer 55 mM NEM-Lösung (in Ethanol) zugegeben, um eine effektive Konzentration von 2,2 mM zu erzielen. Zum anderen "minus" - "plus"-Paar wurden 10 µl einer 55 mM NEM-Biotin-Lösung (in DMSO) zugesetzt, um eine effektive Konzentration von 2,2 mM zu erzielen; es sollte die Zeitabhängigkeit der Alkylierungsreaktion verfolgt werden. Dieser Zeitpunkt wird als Zeitpunkt "0" betrachtet. 50 µl der unterschiedlichen Inkubationen wurden zu bestimmten Zeitpunkten (bis zu 45 min.) entnommen und 10 µl Probenpuffer (50 mM Tris-HCl, pH 6,8, 4 % SDS, 12 % Glycin, 2 % β-Mercaptoethanol, 0,01% SERVA BlueG) zugesetzt. Der molare Überschuß an β-Mercaptoethanol verhinderte die weitere Alkylierung des Rezeptor und inaktivierte restliches NEM bzw. NEM-Biotin im Reaktionsgemisch. Die Reaktionsrate für die Modifizierung der verfügbaren Cystein-Sulphydrylgruppen wurde über deren Biotinylierung verfolgt und mittels nachfolgendem Western-Blot detektiert (Figur 14). Das "minus" - "plus"-Paar, welchem NEM zugegeben worden war, wurde ebenfalls einer PAGE unterzogen und auf PVDF-Membran geblottet. Die Membran wurde über Nacht bei Raumtemperatur getrocknet und sodann mit 20 % Methanol versetzt. hERα wurde direkt auf der PVDF-Membran biotinyliert (1,7 mM NEM-Biotin in TEG-Puffer, 20 min.); siehe Figur 15. Die Reaktion wurde durch wiederholtes Waschen der Membran mit TBS-T (20 mM Tris-HCl, pH 7,6, 137 mM NaCl, 0,05 % TWEEN 20) gestoppt. Zur Verminderung des Hintergrundes wurde die Membran für 1 Stunde geblockt (1 % w/v Casein-Hydrolysat). Der Blockingpuffer wurde anschließend entfernt. Die Membran wurde mit 10 ml einer geeigneten Antikörper-Verdünnung (Anti-Biotin 1 : 2.500 als Peroxidasekonjugat in 20 mM Tris-HCl, pH 7,6, 137 mM NaCl, 0,05 % TWEEN 20, 0,1 % Casein-Hydrolysat) für 1 Stunde inkubiert, 3-malig für 10 min. gewaschen (20 mM Tris-HCl, pH 7,6, 137 mM NaCl, 0,05 % TWEEN 20) und zuletzt mit Wasser gewaschen. Dann wurden jeweils 5 ml ECL-Lösung 1 und 2 zugegeben und der Blot darin 60 Sekunden lang getränkt. Anschließend wurde ein Film aufgelegt. Die Expositionszeit richtete sich nach der Signalstärke.

## Patentansprüche

1. Verfahren zur spezifischen Markierung eines Selenocyst(e)in- und/oder Cyst(e)ingruppen enthaltenden Proteins mit folgenden Schritten:
- mindestens einer Inkubation einer proteinhaltigen Probe mit mindestens einem für Selenocyst(e)in- und/oder Cyst(e)ingruppen spezifischen Modifizierungsreagenz, gefolgt von
- mindestens einer weiteren Inkubation der proteinhaltigen Probe mit mindestens einem für Selenocyst(e)inund/oder Cyst(e)ingruppen spezifischen Markierungsreagenz,
- wobei vor und/oder während und/oder nach mindestens einer der genannten Inkubationen die Zugabe mindestens einer Substanz erfolgt, die mit dem Protein in Wechselwirkung tritt.

2. Verfahren nach Anspruch 1, **dadurch gekennzeichnet, daß** das für Selenocyst(e)in - und/oder Cyst(e)ingruppen spezifische Modifizierungsreagenz diese Gruppen alkyliert.

3. Verfahren nach einem der Ansprüche 1 und/oder 2, **dadurch gekennzeichnet, daß** das für Selenocyst(e)inund/oder Cyst(e)ingruppen spezifische Markierungsreagenz diese Gruppen alkyliert.

4. Verfahren nach mindestens einem der Ansprüche 1 bis 3,**dadurch gekennzeichnet, daß** das Modifizierungsreagenz N-Ethylmaleimid, Dithiothreitol, Dithiorerythriol, β-Mercaptoethanol, Iodacet-amid, Iodacetat, Diamid oder p-CMB ist.

5. Verfahren nach mindestens einem der Ansprüche 1 bis 4, **dadurch gekennzeichnet, daß** das Markierungsreagenz ein Derivat des Modifizierungsreagenzes ist.

6. Verfahren nach mindestens einem der Ansprüche 1 bis 5, **dadurch gekennzeichnet, daß** das Markierungsreagenz lumineszent ist und/oder mindestens einen Luminophor enthält und/oder einen Affinitätsliganden wie Biotin enthält.

7. Verfahren nach mindestens einem der Ansprüche 1 bis 6, **dadurch gekennzeichnet, daß** das Protein enzymatische Aktivität aufweist und/oder ein Rezeptor, insbesondere ein Transmembran-rezeptor, besonders bevorzugt ein 7-Transmembranrezeptor, oder ein löslicher Proteinrezeptor, ist.

8. Verfahren nach mindestens einem der Ansprüche 1 bis 7, **dadurch gekennzeichnet, daß** das Modifizierungsreagenz und/oder das Markierungsreagenz im Überschuß zugesetzt wird.

9. Verfahren nach mindestens einem der Ansprüche 1 bis 8, **dadurch gekennzeichnet, daß** überschüssiges Modifizierungsreagenz vor der Inkubation mit dem Markierungsreagenz entfernt oder inaktiviert wird.

10. Verfahren nach mindestens einem der Ansprüche 1 bis 9, **dadurch gekennzeichnet, daß** die spezifische Markierung des Proteins mittels Western-Blot-Verfahren detektiert wird.

11. Verfahren nach mindestens einem der Ansprüche 1 bis 10, **dadurch gekennzeichnet, daß** die spezifische Markierung des Proteins mittels spektroskopischer Verfahren, insbesondere unter Verwendung eines Detektionssystems auf Basis der konfokalen Fluoreszenzspektroskopie, besonders bevorzugt der Fluoreszenzkorrelationsspektroskopie, und/oder der Nahfeld-spektroskopie detektiert wird.

12. Screeningverfahren zur Bestimmung von Substanzen, die mit einem bevorzugt bekannten Protein in Wechselwirkung treten, wobei das Protein und die Substanz in dem Verfahren nach mindestens einem der Ansprüche 1 bis 11 eingesetzt werden und die Markierung des Proteins detektiert wird.

13. Screeningverfahren zur Bestimmung von Proteinen, die mit einer bevorzugt bekannten Substanz in Wechselwirkung treten, wobei die Proteine und die Substanz in dem Verfahren nach mindestens einem der Ansprüche 1 bis 11 eingesetzt werden und die Markierung des Proteins detektiert wird.

14. Verfahren zum Nachweis der Aktivierung oder Deaktivierung und/oder zur Bestimmung von Konformationsänderungen von Proteinen durch mit diesen in Wechselwirkung tretende Substanzen, wobei die Proteine und die Substanzen in dem Verfahren nach mindestens einem der Ansprüche 1 bis 11 eingesetzt werden und die Markierung des Proteins detektiert wird.

## Claims

1. A method for the specific labeling of a protein containing selenocyst(e)ine and/or cyst(e)ine groups, comprising the following steps:
- at least one incubation of a protein-containing sample with at least one modifying agent specific for selenocyst(e)ine and/or cyst(e)ine groups, followed by
- at least one further incubation of said protein-containing sample with at least one labeling agent specific for selenocyst(e)ine and/or cyst(e)ine groups;
- wherein at least one substance interacting with said protein is added prior to and/or during and/or after at least one of said incubations.

2. The method according to claim 1, **characterized in that** said modifying agent specific for selenocyst(e)ine and/or cyst(e)ine groups will alkylate these groups.

3. The method according to any of claims 1 and/or 2, **characterized in that** said labeling agent specific for selenocyst(e)ine and/or cyst(e)ine groups will alkylate these groups.

4. The method according to at least one of claims 1 to 3, **characterized in that** said modifying agent is N-ethylmaleinimide, dithiothreitol, dithioerythritol, β-mercaptoethanol, iodoacetamide, iodoacetate, diamide or p-CMB.

5. The method according to at least one of claims 1 to 4, **characterized in that** said labeling agent is a derivative of said modifying agent.

6. The method according to at least one of claims 1 to 5, **characterized in that** said labeling agent is luminescent and/or contains at least one luminophor and/or contains an affinity ligand, such as biotin.

7. The method according to at least one of claims 1 to 6, **characterized in that** said protein has enzymatic activity and/or is a receptor, especially a transmembrane receptor, more preferably a 7-transmembrane receptor, or a soluble protein receptor.

8. The method according to at least one of claims 1 to 7, **characterized in that** said modifying agent and/or said labeling agent is added in excess.

9. The method according to at least one of claims 1 to 8, **characterized in that** excess modifying agent is removed or inactivated prior to said incubation with the labeling agent.

10. The method according to at least one of claims 1 to 9, **characterized in that** said specific labeling of the protein is detected by a Western blot method.

11. The method according to at least one of claims 1 to 10, **characterized in that** said specific labeling of the protein is detected by spectroscopical methods, especially using a detection system based on confocal fluorescence spectroscopy, more preferably fluorescence correlation spectroscopy, and/or near-field spectroscopy.

12. A screening method for determining substances which interact with a protein, preferably a known one, wherein said protein and said substance are employed in the method according to at least one of claims 1 to 11 and the labeling of the protein is detected.

13. A screening method for determining proteins which interact with a substance, preferably a known one, wherein said proteins and said substance are employed in the method according to at least one of claims 1 to 11 and the labeling of the protein is detected.

14. A method for the detection of the activation or deactivation and/or for determining conformational changes of proteins using substances which interact with said proteins, wherein said proteins and said substances are employed in the method according to at least one of claims 1 to 11 and the labeling of the protein is detected.

## Revendications

1. Procédé de marquage spécifique d'une protéine contenant des groupes sélénocyst(é)ine et/ou cyst(é)ine, comportant les étapes suivantes :
- au moins une incubation d'un échantillon contenant la protéine avec au moins un réactif de modification spécifique aux groupes sélénocyst(é)ine et/ou cyst(é)ine, suivie
- d'au moins une autre incubation de l'échantillon contenant la protéine avec au moins un réactif de marquage spécifique aux groupes sélénocyst(é)ine et/ou cyst(é)ine,
- dans lequel avant, et/ou pendant, et/ou après au moins une des incubations citées, l'on effectue l'ajout d'au moins une substance qui entre en interaction avec la protéine.

2. Procédé selon la revendication 1, **caractérisé en ce que** le réactif de modification spécifique aux groupes sélénocyst(é)ine et/ou cyst(é)ine alkyle ces groupes.

3. Procédé selon une des revendications 1 ou 2, **caractérisé en ce que** le réactif de marquage spécifique aux groupes sélénocyst(é)ine et/ou cyst(é)ine alkyle ces groupes.

4. Procédé selon au moins une des revendications de 1 à 3, **caractérisé en ce que** le réactif de modification est du N-Ethylmaléimide, du dithiothréitol, du dithiorerythriol, du β-mercaptoéthanol, de l'iodoacétamide, de l'iodoacétate, du diamide ou du p-CMB.

5. Procédé selon au moins une des revendications de 1 à 4, **caractérisé en ce que** le réactif de marquage est un dérivé du réactif de modification.

6. Procédé selon au moins une des revendications de 1 à 5, **caractérisé en ce que** le réactif de marquage est luminescent et/ou contient au moins un luminophore et/ou contient un ligand d'affinité comme la biotine.

7. Procédé selon au moins une des revendications de 1 à 6, **caractérisé en ce que** la protéine a une activité enzymatique et/ou est un récepteur, notamment un récepteur transmembranaire, de préférence un récepteur 7-transmembranaire ou un récepteur protéique soluble.

8. Procédé selon au moins une des revendications de 1 à 7, **caractérisé en ce que** le réactif de modification et/ou le réactif de marquage sont ajoutés en excès.

9. Procédé selon au moins une des revendications de 1 à 8, **caractérisé en ce que** le réactif de modification excédentaire est enlevé ou désactivé avant l'incubation effectuée avec le réactif de marquage.

10. Procédé selon au moins une des revendications de 1 à 9, **caractérisé en ce que** le marquage spécifique de la protéine est détecté au moyen du procédé Western-Blot.

11. Procédé selon au moins une des revendications de 1 à 10, **caractérisé en ce que** le marquage spécifique de la protéine est détecté à l'aide d'une méthode spectroscopique, notamment en utilisant un système de détection basé sur la spectroscopie de fluorescence à focale conjuguée, de préférence sur la spectroscopie de corrélation de fluorescence, et/ou sur la spectroscopie de champ proche.

12. Procédé de screening pour l'analyse de substances qui interagissent avec une protéine connue de préférence, dans lequel on utilise la protéine et la substance dans le procédé selon au moins une des revendications de 1 à 11 et l'on détecte le marquage obtenu de la protéine.

13. Procédé de screening pour l'analyse de protéines qui interagissent avec une substance connue de préférence, dans lequel l'on utilise les protéines et la substance dans le procédé selon au moins une des revendications de 1 à 11 et l'on détecte le marquage obtenu de la protéine.

14. Procédé de mise en évidence de l'activation ou de l'inactivation de protéines et/ou de détermination des modifications de conformation de protéines par des substances exerçant une interaction avec celles-ci, dans lequel l'on utilise les protéines et les substances dans le procédé selon au moins une des revendications de 1 à 11 et l'on détecte le marquage obtenu de la protéine.
